(19)

Europäisches
Patentamt
European
Patent Office
Office européen
des brevets

(11) **EP 2 434 411 A1**

(12) **EUROPEAN PATENT APPLICATION**

(43) Date of publication:
**28.03.2012 Bulletin 2012/13**

(51) Int Cl.:
**G06F 17/18** $^{(2006.01)}$

(21) Application number: **10180086.0**

(22) Date of filing: **27.09.2010**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(71) Applicant: **Qlucore AB
223 70 Lund (SE)**

(72) Inventor: **Fontes, Magnus
SE-224 75, LUND (SE)**

(74) Representative: **Ström & Gulliksson AB
Box 4188
203 13 Malmö (SE)**

(54) **Computer-implemented method for analyzing multivariate data**

(57)    A computer-implemented method for analyzing multivariate data comprising a plurality of samples of each of a plurality of measurement variables is disclosed. The method comprises, for a first subset $\phi_A(X)$ of the multivariate data $X$, determining (110) a first projection score related to the first subset. Furthermore, the method comprises, for a second subset $\phi_B(X)$ of the multivariate data $X$, determining (120) a second projection score related to the second subset. Moreover, the method comprises, comparing (130) the first and the second projection score for determining which one of the first and the second subset provides the most informative representation of the multivariate data, which is defined as the one of said subsets having the highest related projection score. A definition of the projection score is also provided.

Fig. 10

**Description**

**Technical Field**

The present invention relates to a method for analyzing multivariate data, in particular multivariate technical measurement data.

**Background**

Statistical analyses of measurement data is important in many technical fields. Such measurement data may be multivariate, involving a relatively large number of measured variables (such as but not limited to in the order of $10^4$ — $10^{10}$), and involve a relatively large number of samples (such as but not limited to in the order of $10$ — $10^6$) of each variable. Nonlimiting examples of technical fields where such measurement data may arise is astronomy, meteorology, and biotech. In meteorology, the measured variables may e.g. include temperatures, air pressures, wind velocities, and/or amounts of precipitation, etc., at various locations. In the biotech field, the measured variables may e.g. be expression levels of genes or proteins.

A technical problem associated with the analysis of such relatively large amounts of measurement data is that currently available computers lack sufficient hardware resources for performing various parts of the analyses in a timely manner, if at all possible.

Some parts of an analysis, such as performing a t-test or ANOVA (analysis of variance) test, may be performed relatively efficiently on existing hardware. However, in order to evaluate the informativeness of a given test, general criteria that can be efficiently implemented on existing hardware are needed. The goal may be to identify "hidden" structures in and/or relationships between the measured samples of the measurement variables. This process may include projecting the measurement data onto a subspace of the measured variables in order to reduce the degrees of freedom. Thereby, particularly relevant variables or combination of variables are selected for further analysis, whereas other variables or combinations thereof that are less relevant may be omitted. Such a projection naturally involves a loss of information, and it is desirable to keep this loss as small as possible, e.g. select the variables or combinations thereof that are most informative. Depending e.g. on the amount of data, such identification of hidden structures and/or relationships may take excessive time to perform on existing computer hardware, or may in some cases not be possible to perform at all. Therefore, in this respect, technical considerations, taking into account the limited hardware resources of currently available computers, are needed for developing improved methods that can be executed on such currently available computers in a reasonable amount of time.

**Summary**

In order to reduce the above-identified technical problem associated with insufficient computer hardware resources, the inventor has developed a metric or a measure, in the following referred to as the projection score, that can be used for comparing the informativeness in different subsets of multivariate data. The projection score can be relatively rapidly computed on relatively modest computer hardware, thereby facilitating relatively rapid identification of information-bearing structures (in a statistical sense) in and/or relationships between samples of measured variables.

According to a first aspect, there is provided a computer-implemented method for analyzing multivariate data comprising a plurality of samples of each of a plurality of variables. The method comprises, for a first subset $\phi_A(X)$ of the multivariate data $X$, determining a first projection score related to the first subset. The method further comprises, for a second subset $\phi_B(X)$ of the multivariate data $X$, determining a second projection score related to the second subset. Moreover, the method comprises comparing the first and the second projection score for determining which one of the first and the second subset provides the most informative representation of the multivariate data, which is defined as the one of said subsets having the highest related projection score. The projection score related to a given submatrix $\phi_m(X)$ of the multivariate data matrix $X$ is defined as

$$\sigma\big(\phi_m(X), S, \mathscr{P}_{\phi_m(X)}\big) = \frac{g(\Lambda_{\phi_m(X)}, S)}{\mathbb{E}_{\mathscr{P}_{\phi_m(X)}}[g(\Lambda_{\phi_m(X)}, S)]},$$

wherein

- $\phi_m(X)$ is a $K_m$ x N matrix of rank $r$ comprising measurement data of the subset, wherein $K_m$ and N are integers representing the number of variables and the number of samples, respectively;

- $g(\Lambda_{\phi m(X)}, S)$ is selected from the set

$$\mathcal{G} = \{h \circ \alpha_q \colon \mathbb{R} \to \mathbb{R};$$

$h$ is increasing *and* $q \geq 1\}$ for

$$\alpha_q\left(\Lambda_{\phi_m(X)}, S\right) = \frac{\sum_{k \in S} \lambda_k^q\left(\phi_m(X)\right)}{\sum_{k=1}^{r} \lambda_k^q\left(\phi_m(X)\right)}$$

- $\lambda_1 \geq \lambda_2 \geq ... \geq \lambda_r > 0$ are the singular values of $\phi_m(X)$;
- S is a set of indices i representing principal components of $\phi_m(X)$ onto which the data in $\phi_m(X)$ is projected;and

$$-\mathbb{E}_{\mathcal{P}_{\phi_m(X)}}\left[g(\Lambda_{\phi_m(X)}, S)\right]$$

is the expectation value, or estimate thereof, of $g(\Lambda_{\phi m(X)}, S)$ for a matrix probability distribution $P_{\phi m(x)}$.

The method may comprise, for each of one or more additional subsets of the multivariate data, determining a projection score related to that subset. Furthermore, the method may comprise comparing the projection scores related to the one or more additional subsets of the multivariate data and the first and the second projection score for determining which one of the first and the second subset provides the most informative representation of the multivariate data, which is defined as the one of said subsets having the highest related projection score.

The method may comprise selecting one of the subsets for further statistical analysis based on the comparison of the first and the second projection score.

The comparing of the projection scores may be part of a statistical hypothesis test.

The multivariate data may be technical measurement data, such as astronomical measurement data, meteorological measurement data, and/or biological measurement data.

According to a second aspect, there is provided a computer program product comprising computer program code means for executing the method according to the first aspect when said computer program code means are run by an electronic device having computer capabilities.

According to a third aspect, there is provided a computer readable medium having stored thereon a computer program product comprising computer program code means for executing the method according to the first aspect when said computer program code means are run by an electronic device having computer capabilities.

According to a fourth aspect, there is provided a computer configured to perform the method according to the first aspect.

According to a fifth aspect, there is provided a method of determining a relationship between a plurality of physical and/or biological parameters. The method according to this fifth aspect comprises obtaining multivariate data representing multiple samples of observed values of said plurality of parameters and analyzing the multivariate data using the method according to the first aspect.

Further embodiments of the invention are defined in the dependent claims.

It should be emphasized that the term "comprises/comprising" when used in this specification is taken to specify the presence of stated features, integers, steps, or components, but does not preclude the presence or addition of one or more other features, integers, steps, components, or groups thereof.

**Brief Description of the Drawings**

Further objects, features and advantages of embodiments of the invention will appear from the following detailed description, reference being made to the accompanying drawings, in which:

Figs. 1-9 show plotted data according to examples;
Fig. 10 is a flowchart of a method according to an embodiment of the present invention; and

Fig. 11 schematically illustrates a computer and a computer-readable medium.

**Detailed Description**

Embodiments of the present invention concerns evaluation criteria for subset selection from multivariate datasets , where possibly the number of variables is much larger than the number of samples, with the aim of finding particularly informative subsets of variables and samples. Such data sets are becoming increasingly common in many fields of application, for example in molecular biology where different omics data sets containing tens of thousands of variables measured on tens or hundreds of samples are now routinely produced. In accordance with some embodiments and examples, so called unsupervised learning and/or visualization through Principal Components Analysis (PCA) are considered. PCA creates a low-dimensional sample representation that encodes as much as possible of the variance in the original data set by projecting onto linear combinations of the original variables, called principal components. However, conventionally, the principal components are linear combinations of all the measured variables, which may render them difficult to interpret if the number of variables is very large. Furthermore, it is reasonable to believe that only a subset of the variables are involved, to a relevant extent, in many typical processes of interest. If the data matrix contains a large number of uninformative variables, just adding more or less random variation, the clarity and interpretability of the obtained low-dimensional sample configuration may be compromised. Hence, the inventor has realized that variable selection can be useful in the unsupervised learning and/or visualization context.

For different types of high-throughput Omics data, e.g. expression levels for mRNA data corresponding to genes, a variable selection step may be performed prior to analysis by applying a variance filter, removing genes which are almost constant across all samples in the data set. However, there exists no widely used stopping criterion for determining an appropriate variance threshold to use as inclusion criterion, leading to ad hoc chosen thresholds. Furthermore, routinely filtering away variables with low variance may exclude potentially informative variables. Other types of variable selection may also be used for pre-processing of microarray data, such as including only variables which are significant with respect to a given statistical test. Also in these cases, a tuning parameter (the significance level) must be decided upon. Ideally, it should be chosen to provide a good trade-off between the number of false discoveries and the number of false negatives.

In accordance with embodiments of the present invention, a concept referred to as "projection score" is introduced. The projection score can be seen as a measure of the informativeness of a PCA configuration obtained from a subset of the variables of a given multivariate data set.

The projection score can be used for example to compare the informativeness of configurations based on different subsets of the same variable set, obtained by variance filtering with different variance thresholds. The optimal projection score is then obtained for the variance threshold which provides the most informative sample configuration. The calculation of the projection score is based on the singular values of the data matrix, and essentially compares the singular values of observed data to the expected singular values under a null model, which can be specified e.g. by assuming that all variables and samples, respectively, are independent. The projection score can be useful as a stopping criterion for variance filtering preceding PCA. Furthermore, the projection score can be used to provide a suitable significance threshold for certain statistical tests. Moreover, the projection score can be used to detect sparsity in the leading principal components of a data matrix.

Let $X = [x_1,...,x_N] \in \mathbb{R}^{p \times N}$ be a given matrix with rank $r$, containing N samples of $p$ random variables. The notation $r$ is used throughout this description to denote the rank of various matrices. However, this does not imply that the matrices all have the same rank. Instead, $r$ can bee seen as a variable parameter that can adopt different values for different matrices (depending on the ranks of the different matrices). So called principal components analysis (PCA) reduces the dimensionality of the data by projecting the samples onto a few uncorrelated latent variables encoding as much as possible of the variance in the original data. Assuming that each variable is meancentered across the samples, the empirical covariance matrix (scaled by N) is given by C = $XX^T$. The covariance matrix is positive semi-definite with rank $r$, and hence, by the spectral theorem, we have a decomposition

$$XX^T V = V \Lambda^2$$

$V = [v_1,..., v_r]$ is a $p$ x r matrix such that $V^T V = I_r$, where $I_r$ is the $r$ x $r$ identity matrix. Furthermore, A = $diag$ ($\lambda_1$ $(X),...,\lambda_r(X)$) is the $r$ x $r$ diagonal matrix having the the positive square root of the non-zero eigenvalues $of XX^T$ (i.e. the singular values of X) on the diagonal. The orthonormal columns of V are the principal components, and the coordinates of the samples in this basis are given by U = $X^T V$. A lower-dimensional sample configuration is obtained by selecting the columns of U with index in a specified subset S c {1, ..., r}. Each row of this matrix then represents one sample in an |S|-dimensional space. According to some embodiments of the present invention, an objective is to find particularly informative such sample configurations for a given choice of S, by including only a subset of the original variables with non-zero weights in the principal components.

The first principal component is the linear combination of the original variables which has the largest variance, and the variance is given by $\lambda_1^2$. Similarly, the second principal component has the largest variance among linear combinations which are uncorrelated with the first component. The n:th principal component has a variance given by $\lambda_n^2$. Given a subset S c {1, ..., r}, the fraction of the total variance encoded by the principal components with index in S is consequently

$$\alpha_2(\Lambda_X, S) = \frac{\sum_{k \in S} \lambda_k^2(X)}{\sum_{k=1}^r \lambda_k^2(X)}$$

More generally, the $L^q$ norm $(q \geq 1)$ can be used to measure the information content (where the $L^2$ norm represented above with $\alpha_2$ is a special case), giving a measure of the explained fraction of the information content as

$$\alpha_q(\Lambda_X, S) = \frac{\sum_{k \in S} \lambda_k^q(X)}{\sum_{k=1}^r \lambda_k^q(X)}$$

According to some embodiments of the present invention, S is chosen as S = {1,2} or S = {1,2,3}. Such a selection of S allows for a relatively easy visualization of the resulting sample configurations. A specific choice of S effectively indicates which part of the data is to be considered as the "signal" of interest, and the rest is in some sense considered "irrelevant". The interpretation of $\Sigma_{k \in S} \lambda_k^2(X)$ as the variance captured by the principal components with index in S implies that it can be used as a measure of the amount of information captured in the corresponding |S|-dimensional sample configuration. However, for a given S, the expected value of this statistic depends heavily on the underlying distribution of the matrix X. In accordance with some embodiments of the present invention, this is taken into account in order to obtain a more suitable measure of the "informativeness" of a given low-dimensional sample configuration. Therefore, in accordance with these embodiments, the projection score is introduced in accordance with the definition below.

Definition (Projection score): Let $X = [x_1, ..., x_N] \in \mathbb{R}^{pxN}$ be a matrix with rank r. For a given matrix probability distribution $P_x$, a subset $S \subset \{1, ..., r\}$, and a function $g: \mathbb{R}_+^r \times 2^{\{1,...,r\}} \to \mathbb{R}$, the projection score $\sigma(X, S, g, P_x)$ is defined as

$$\sigma(X, S, g, \mathcal{P}_X) = \frac{g(\Lambda_X, S)}{\mathbb{E}_{\mathcal{P}_X}[g(\Lambda_X, S)]}$$

where $\Lambda_X = ((X), ..., \lambda_r(X))$ is the vector of length r containing the singular values of X in decreasing order. $\mathbb{E}_{\mathcal{P}_X}[g(\Lambda_X, S)]$ denotes the expectation value (or estimate thereof) of g ($\Lambda_x$, S) for the given matrix probability distribution $P_x$.

In accordance with some embodiments of the present invention, g is chosen from a family of functions given by

$G = \{h \circ \alpha_q \colon \mathbb{R} \to \mathbb{R} \; ; \; h$ is increasing *and* $q \geq 1\}$ $\mathbb{E}_{\mathcal{P}_X}[g(\Lambda_X, S)]$ may e.g. be estimated using known methods based on permutation and/or randomization.

**Example embodiments**

Below, some example embodiments utilizing the projection score are presented. In these examples, it is e.g. shown how the projection score can be used to compare the informativeness of sample configurations obtained by applying PCA to different submatrices of a given matrix $X$. We define functions $\phi_m \colon \mathbb{R}^{p \times N} \to \mathbb{R}^{K_m \times N}, m = 1,..., M$ such that $\phi_m(X)$ is a submatrix of $X$ with $K_m$ rows. In other words, each m selects a subset of the variables in the original matrix. For example, we may define $\phi_m(X)$ as the submatrix of $X$ consisting of the $K_m$ rows corresponding to the variables with highest variance. Given a null distribution $P_{\phi m(X)}$ for each $\phi_{m(X)}$, we can calculate the projection score $\sigma(\phi_m(X), S, g, P_{\phi m(X)})$. For fixed S and $g \in$ G (where G is defined as above), a submatrix $\phi_A(X)$ is considered to be more informative than a submatrix $\phi_B(X)$ if $\sigma(\phi_A(X), S, g, P_{\phi A(X)}) \geq \sigma(\phi_B(X), S, g, P_{\phi B(x)})$

Following the definition of $\sigma(X,S,g,P_x)$ above, $\sigma(\phi_m(X), S, g, P_{\phi m(X)})$ (where $\phi_m(X)$ is any of the submatrices of $X$, e.g. $\phi_A(X)$ or $\phi_B(X)$) is given by

$$\sigma\big(\phi_m(X), S, \mathcal{P}_{\phi_m(X)}\big) = \frac{g(\Lambda_{\phi_m(X)}, S)}{\mathbb{E}_{\mathcal{P}_{\phi_m(X)}}[g(\Lambda_{\phi_m(X)}, S)]}$$

$\phi_m(X)$ is a $K_m \times N$ matrix of rank *r* comprising measurement data of a subset of the matrix $X$, and $K_m$ and N are integers representing the number of variables and the number of samples, respectively. The function $g(\Lambda_{\phi m(X)}, S)$ is selected from the set

$G = \{h \circ \alpha_q \colon \mathbb{R} \to \mathbb{R} \; ; \; h$ is increasing and $q \geq 1\}$
for

$$\alpha_q\big(\Lambda_{\phi_m(X)}, S\big) = \frac{\sum_{k \in S} \lambda_k^q\big(\phi_m(X)\big)}{\sum_{k=1}^r \lambda_k^q\big(\phi_m(X)\big)}$$

$\lambda_1 \geq \lambda_2 \geq \cdots \geq \lambda r > 0$ are the singular values of $\phi_m(X)$, S is a set of indices i representing principal components of $\phi_m(X)$ onto which the data in $\phi_m(X)$ is projected, and

$$\mathbb{E}_{\mathcal{P}_{\phi_m(X)}}[g(\Lambda_{\phi_m(X)}, S)]$$

is the expectation value (or estimate thereof) of $g(\Lambda_{\phi(X)}, S)$ for the matrix probability distribution $P_{\phi m(X)}$.

The null distribution $P_{\phi m(X)}$, for matrices of dimension $K_m \times N$, can be defined in different ways. One relatively simple way is to assume that every matrix element $x_{ij}$ is drawn independently from a given probability distribution, e.g. $x_{ij} \in N(0,1)$ for $i = 1, ..., K_m$ and $j = 1,...,N$. Then, the highest projection score is obtained for the submatrix whose singular values deviate most, in a sense given by the chosen $g \in$ G, from what would be expected if all matrix elements were independent standard normally distributed variables. However, even if the original data set consists of independent normally distributed variables, this is not in general true after applying $\phi_m(X)$. Hence, even a submatrix obtained by filtering independent normally distributed variables may be far from the null distribution defined this way.

According to example embodiments presented in this description, $P_x$ is defined by assuming that $X$ consists of N independent samples of *p* independent variables with some distribution. The null distribution of each variable in $\phi_m$

($X$) is then defined by truncation of the corresponding distribution obtained from $P_x$, with respect to the features of $\phi_m$. For example, samples X*d, $d$ = 1, ..., D (for some integer D) can be generated from $P_x$ by permuting the values in each row of $X$ independently. For each $X^{*d}$, $g(\Lambda_{X^{*d}}, S)$ can be computed. The expectation value of $g(\Lambda_{X^{*d}}, S)$ under the probability distribution $P_x$ can then be estimated according to

$$\mathbb{E}_{\mathcal{P}_X}[g(\Lambda_X, S)] = \frac{1}{D}\sum_{d=1}^{D} g(\Lambda_{X^{*d}}, S)$$

Similarly, the expectation value of $g(\Lambda_{\phi_m(X)}, S)$ may, according to some embodiments, be estimated by repeated permutation of the values in each row of $X$ followed by application of $\phi_m$ to the permuted matrix. Hence, according to some embodiments, the expectation value of $g(\Lambda_{\phi_m(X)}, S)$ may be estimated as

$$\mathbb{E}_{\mathcal{P}_{\phi_m(X)}}[g(\Lambda_{\phi_m(X)}, S)] = \frac{1}{D}\sum_{d=1}^{D} g(\Lambda_{\phi_m(X^{*d})}, S)$$

As discussed above, $S$ may be selected to admit relatively simple visualization. Several methods have also been proposed to determine the "true" dimensionality of a data set, i.e. the number of principal components that should be retained. When the number of variables is decreased, the true dimensionality of the resulting data set may change. We say that a submatrix $\phi_m(X)$ supports a given S if the variance accounted for by each of the principal components of $\phi_m(X)$ with index in S is large enough. More specifically, we estimate the distribution of $\lambda_k^2(\phi_m(X))$ for each $k \in S$ under the probability distribution $P_{\phi_m(X)}$. If the estimated probability of obtaining a value of $\lambda^2_k(\phi_m(X))$ at least as large as the observed value is less than some threshold, such as but not limited to 5%, for all $k \in S$, we say that the submatrix $\phi_m(X)$ *supports S.* In examples presented herein, said threshold is assumed to be 5%. The null distribution of $\lambda_k^2(\phi_m(X))$ may, according to some embodiments of the present invention, be estimated from the singular values of the submatrices $\phi_m(X^{*d})$. Permutation methods similar to this approach, comparing some function of the singular values between the observed and permuted data, have been used and validated in several studies to determine the number of components to retain in PCA.

The number of variables ($K_m$) to be included in each step can be determined by setting threshold levels on an underlying statistic in the observed matrix $X$. The same number of variables ($K_m$) is then included in each of the permuted matrices. For example, one can successively include all variables with variance greater than 1%, 2%, ... of the maximum variance among all variables.

Note that that $X$, in some cases, may comprise data from a single original (multivariate) dataset. However, in other cases, $X$ may comprise data from two or more (possibly unrelated) original datasets. For example, $X$ may be seen as an aggregation of said two or more original datasets. Thus, the projection score can not only be used for comparing the informativeness of different subsets of a single original dataset, but also for comparing the informativeness of different (possibly unrelated) original datasets (or subsets thereof), for example by letting a first subset $\phi_1(X)$ only comprise data from one of the original datasets and second subset $\phi_2(X)$ only comprise data from another one of the original datasets.

### *Example application 1: Variance filtering*

In this section, we show how the projection score may be used according to some embodiments as a stopping criterion for variance filtering. For a given set of variance thresholds $\theta_m$, we let $\phi_m(X)$ contain all variables with variance exceeding $\theta_m$. $K_m$ is taken as the number of rows of $\phi_m(X)$. To obtain a relatively low-dimensional sample configuration that reflects the correlation structure between the variables of the data set instead of the individual variances, the principal components may be extracted from standardized data matrices, where each variable is mean-centered and scaled to unit variance. This approach is used in the examples presented herein. Hence, the principal components will be eigenvectors of the correlation matrix rather than the covariance matrix. In the illustrative examples presented herein, the data matrices are permuted D = 100 times. However, this number is by no means intended to be limiting. Furthermore, in these examples, the function $g \in G$ is chosen as

$$g(\Lambda_X, S) = \frac{\sum_{k \in S} \lambda_k^2(X)}{\sum_{k=1}^{r} \lambda_k^2(X)}$$

$$(1)$$

which can be interpreted as the fraction of variance related to the extracted principal components. This choice of $g \in$ G is not intended to be limiting, and other choices of $g \in$ G may well be used in other embodiments.

*Example 1 (Synthetic data):*

As an illustrative example, we let

$$\mu_{1j} = \begin{cases} -0.5 & \text{if} \quad 1 \le j \le 50 \\ +0.5 & \text{if} \quad 51 \le j \le 100 \end{cases}$$

and generate a synthetic data set with 1000 variables and 100 samples by letting

$$x_{ij} \in \begin{cases} \mathcal{N}(\mu_{1j}, \sigma_1) & \text{if} \quad 1 \le i \le 150 \\ \mathcal{N}(0, 0.5) & \text{if} \quad 151 \le i \le 1000 \end{cases}$$

The only informative structure in the data is contained in the first 150 variables, discriminating between two groups of 50 samples. By varying $\sigma_1$, we obtain data sets with difference variance properties. By choosing $\sigma_1 = 0.5$, the informative variables and the non-informative variables have comparable variances. By choosing $\sigma_1 = 0.2$, the informative variables obtain a lower variance than the non-informative variables. By choosing $\sigma_1 = 0.8$, the informative variables are also those with highest variance.

For this example, we take S = {1}, since no other choice of *S* is supported by any submatrix . This is also consistent with the structure of the data matrix. The highest projection scores are obtained by including, respectively, the 921 ($\sigma_1 = 0.5$), 1000 ($\sigma_1 = 0.2$) or 140 ($\sigma_1 = 0.8$) variables with highest variance. The projection score correctly indicates that when $\sigma_1 = 0.2$, the informative structures in the data are actually related to the variables with lowest variance, and hence all variables should be included to obtain an informative projection. Note that the association between the variables within each sample group is very strong when $\sigma_1 = 0.2$. If the variables with lowest variance had been routinely filtered out in this example, we would lose the informativeness in the data. It can also be noted that when the number of variables is less than a certain threshold (approximately 850) in the case $\sigma_1 = 0.2$, not even S = {1} is supported by the data since we have filtered out all informative variables. When $\sigma_1 = 0.8$, the highly varying variables are also the informative ones, and the optimal number of variables is 140, close to the 150 which were simulated to be discriminating.

*Example 2 (cell culture data):*

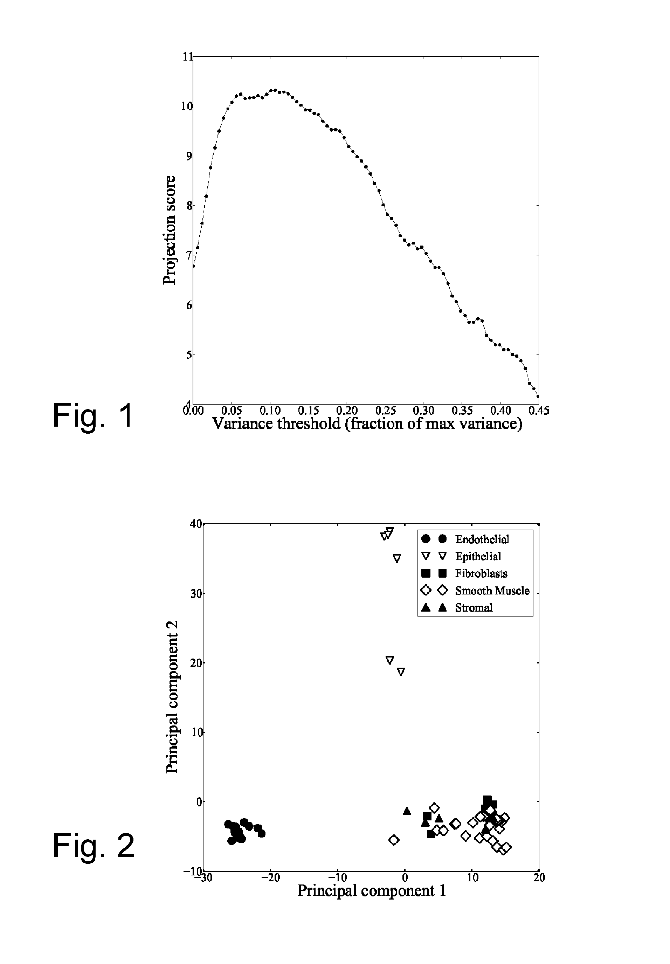

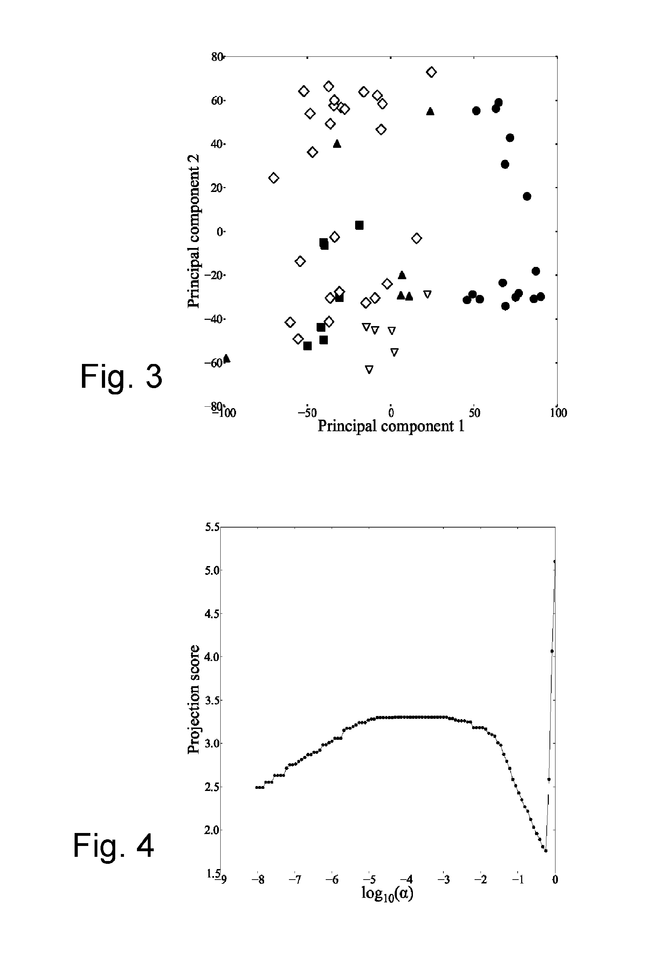

The data set used in this example comes from a microarray study of gene expression profiles from 61 normal human cell cultures, taken from five cell types in 23 different tissues or organs. The data set was downloaded from the National Center for Biotechnology Informations (NCBI) Gene Expression Omnibus (GEO, http://www.ncbi.nlm.nih.gov/geo/, data set GDS1402). The original data set contains 19,664 variables. We remove the variables containing missing values (2,741 variables) or negative expression values (another 517 variables), and the remaining expression values are $\log_2$-transformed. We use S = {1,2}, and hence obtain an informative two-dimensional sample configuration. Fig. 1 shows the projection score as a function of the variance threshold (fraction of the maximal variance) used as inclusion criterion. The optimal projection score is obtained when 656 variables are included, equivalent to a variance threshold at 10.7% of the maximal variance among the variables. Fig. 2 shows the sample configuration corresponding to the optimal projection score, and Fig. 3 shows the sample configuration obtained by projection onto the first two principal components from the original data matrix, containing all 16,406 variables. Fig. 2 shows that applying PCA to the data set consisting of the 656 variables with highest variance in the data set provides a two-dimensional sample configuration where two of the sample groups can be extracted. It can also be noted that in this example, apparently much of the structure in the data is related to the variables with the highest variance.

### _Example application 2: Filtering with respect to the association with a given response_

In example embodiments presented in this section, we let $\phi_m(X)$ consist of the $K_m$ variables that are most highly related to a given response variable. In the studied examples the response variable indicates the partition of the samples into different groups. Given such a partition, we calculate the F-statistic, contrasting all these groups, for each variable. For a given set of significance thresholds $\alpha_m$, we let $\phi_m(X)$ include all variables which are significantly related to the response at the level $\alpha_m$. Also in the example embodiments presented in this section, we choose the function $g$ given by (1). The choice of S is guided by the underlying test statistic. If we contrast only two groups, we do not expect the optimal variable subset to support more than a one-dimensional sample configuration, and hence we choose S = {1} in this case. To obtain an informative higher-dimensional configuration in this case, also variables not related to the discrimination of the two groups would need to be included. When contrasting more than two groups, the choice of S is more complicated. This is because the variables with the highest F-score may in this case very well characterize many different samples, not all of which can simultaneously be accurately visualized in low dimension.

_Example 3 (Synthetic data):_

According to this example, we simulate a data matrix containing two group structures. The data set consists of 40 samples which are divided into four consecutive groups of 10 samples each, denoted a, _b,_ c, d. We define

$$\mu_{1j} = \begin{cases} -2 & \text{if} \quad j \in a \cup b \\ +2 & \text{if} \quad j \in c \cup d \end{cases}$$

and

$$\mu_{2j} = \begin{cases} -1 & \text{if} \quad j \in a \cup c \\ +1 & \text{if} \quad j \in b \cup d \end{cases}$$

The data matrix is then generated by letting

$$x_{ij} \in \begin{cases} \mathcal{N}(\mu_{1j}, 1) & \text{if} \quad 1 \leq i \leq 200 \\ \mathcal{N}(\mu_{2j}, 1) & \text{if} \quad 201 \leq i \leq 250 \\ \mathcal{N}(0,1) & \text{if} \quad 251 \leq i \leq 1000 \end{cases}$$

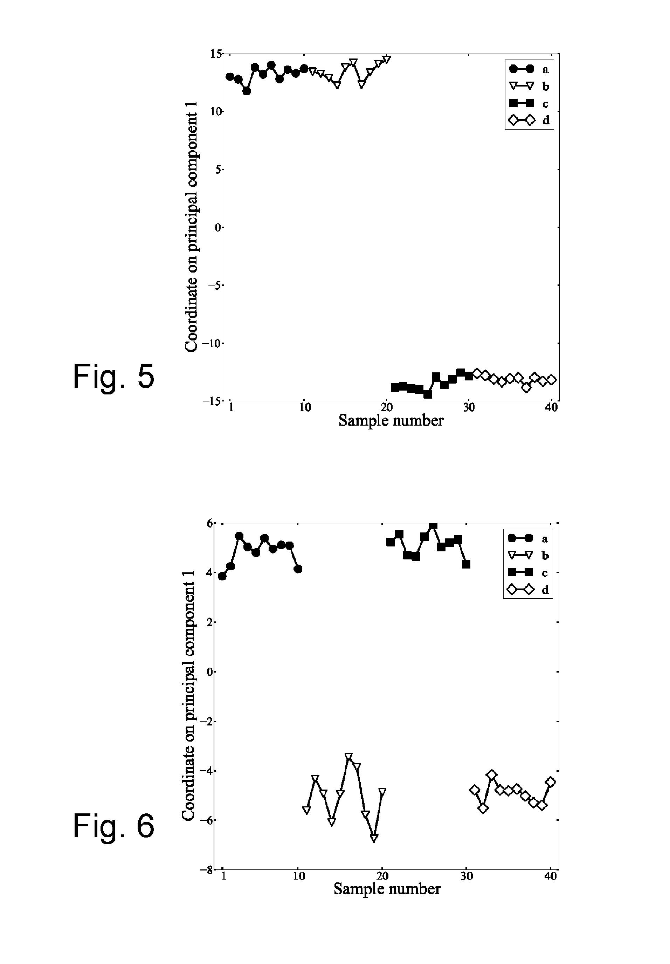

We perform an F-test contrasting $a \cup c$ and $b \cup d$ and order the variables by their F-statistic for this contrast. In this case, since we compare only two groups, we are essentially searching for a one-dimensional separation, so we choose S = {1}. The data set contains one very strong factor, encoded by the first 200 variables, and one weaker factor, the one we are interested in, which is related to the next 50 variables. Fig. 4 shows the projection score for different threshold levels on the significance level. From Fig. 4, it can be deduced that the optimal projection score is obtained by including all 1000 variables, corresponding to $\log_{10}(\alpha) = 0$. However, the first principal component in this case discriminates between $a \cup b$ and $c \cup d$, as can be seen in Fig. 5. The behavior of the projection score indicates that the projection based on the entire variable set is more informative than any projection which is based only on variables related to the weaker factor. If we decrease the significance threshold, we reach a local maximum at 50 variables. Projecting onto the first principal component based only on these variables clearly discriminates between $a \cup c$ and $b \cup d$, as shown in Fig. 6. This example indicates that suggests that the projection score may be useful for obtaining a significance threshold which gives an good trade-off between the false discovery rate and the false negative rate, and that it can be informative to study not only the global maximum of the projection score curve, but also local maxima. Hence, some embodiments of the present invention comprises searching for and/or locating one or more local maxima of the projection score.

*Example 4 (Cell culture data):*

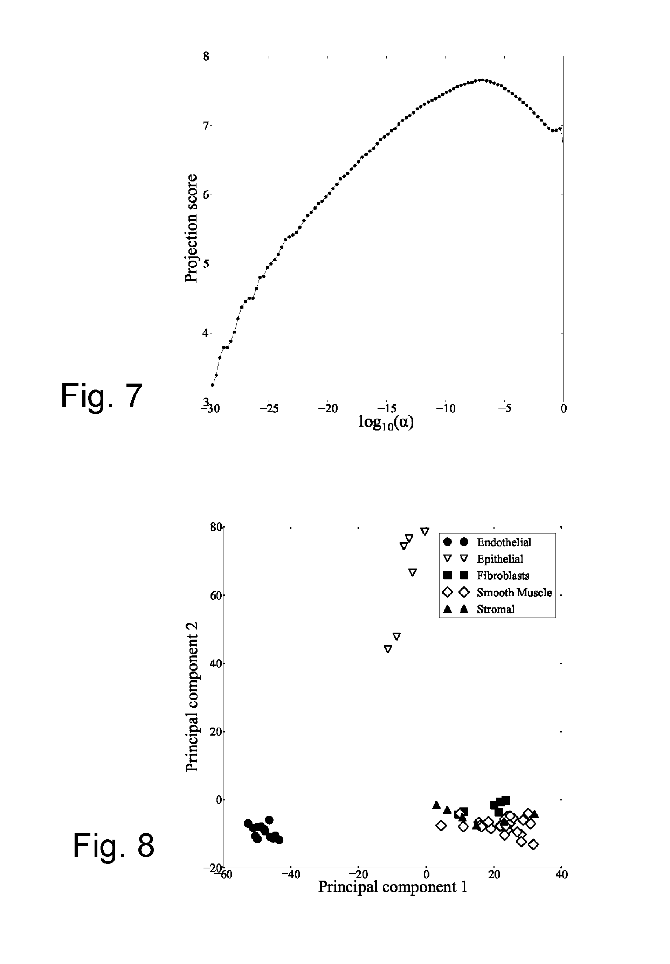

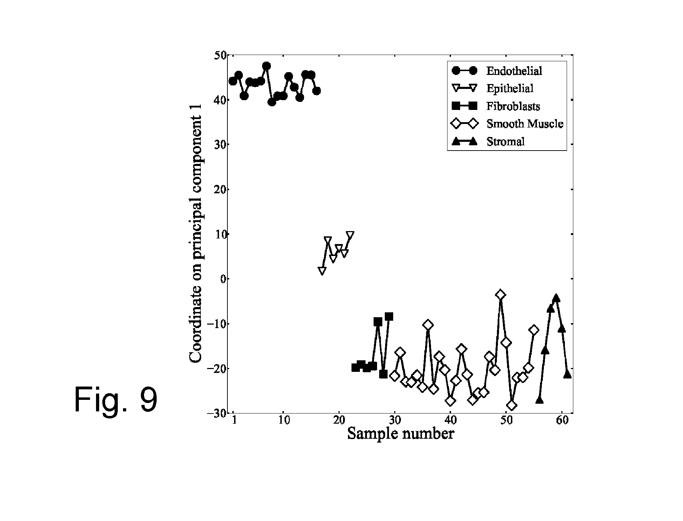

In this example, we filter the cell culture data (same data as in Example 2), letting $\phi_m(X)$ consist of the variables having the highest value of the F-statistic contrasting all five sample groups and using S = {1,2}. Fig. 7 shows the projection score as a function of the significance threshold. Fig. 8 shows the projection corresponding to the best projection score (obtained for 2326 variables). The sample configuration based on all variables is the same as in a previous example and is illustrated in Fig. 3. As can be seen in Fig. 8, the 2326 included variables essentially characterize two of the sample groups. It can also be noted from this Fig. 8 that already the first principal component could potentially yield the same three sample clusters. Optimizing the projection score using instead S = {1} gives an optimal variable set consisting of 1770 variables. The projection of the samples onto the extracted component is given in Fig. 9. Hence, by choosing S = {1} in this example, we extract variables which have a characteristic expression in the endothelial cell samples, and to a lesser extent also in the epithelial cell samples. Choosing S = {1,2}, we include an additional set of variables, which are specific for the epithelial cell samples. We can try also S = {1,2,3} to possibly extract additional informative variables, characterizing another sample group. In this case, when the number of included variables is less than 2000 the, three-dimensional configuration is no longer supported, indicating that the 2,000 variables with highest F-score essentially provides a two-dimensional sample configuration. The most informative configuration for S = {1,2,3} is obtained by including almost all variables, corresponding to a significance threshold of $\alpha$ = 0.489 (data not shown). This suggests that it is not possible (for this particular data set) to obtain an informative, truly three-dimensional configuration based only on variables with a high F-score.

According to embodiments of the present invention, there is thus provided a computer-implemented method for analyzing technical measurement data (e.g. measurement data relating to any physical and/or biological process and/or phenomenon) comprising a plurality of samples of each of a plurality of measurement variables. The method comprises, for a first subset $\phi_A(X)$ of the measurement data $X$, determining a first projection score related to the first subset. Furthermore, the method comprises, for a second subset $\phi_B(X)$ of the measurement data $X$, determining a second projection score related to the second subset. Moreover, the method comprises comparing the first and the second projection score for determining which one of the first and the second subset provides the most informative representation of the measurement data, which is defined as the one of said subsets having the highest related projection score.

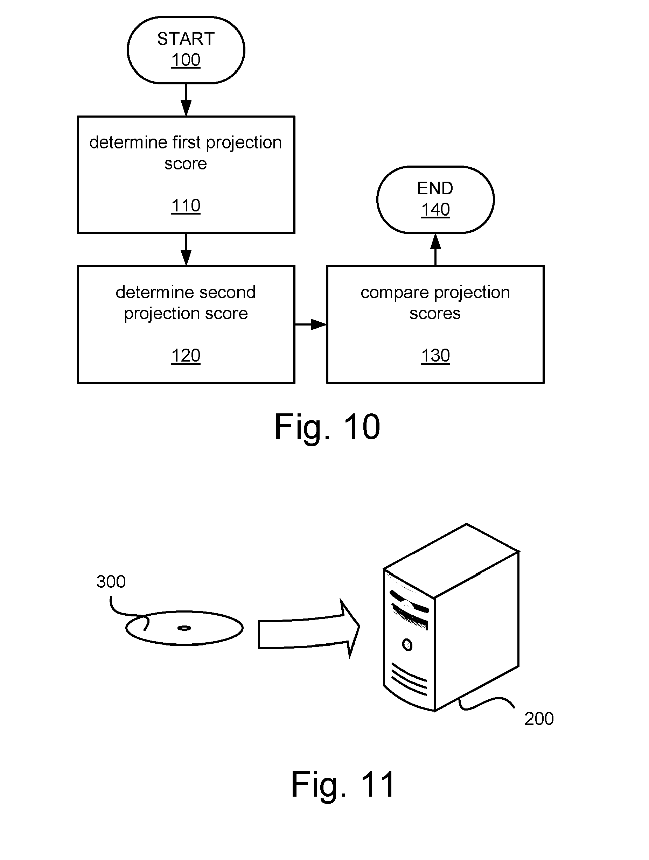

An embodiment of the method is illustrated with a flowchart in Fig. 10. The operation is started in step 100. In step 110, the first projection score is determined. In step 120, the second projection score is determined. A comparison of the first and second projection score is performed in step 130, and the operation is then ended in step 140.

In some embodiments, the method may further comprise, for each of one or more additional subsets (i.e. in addition to $\phi_A(X)$ and $\phi_B(X)$) of the measurement data, determining a projection score related to that subset. Such embodiments may further comprise comparing the projection scores related to the one or more additional subsets of the measurement data and the first and the second projection score for determining which one of these subsets provides the most informative representation of the measurement data, which is defined as the one of said subsets having the highest related projection score.

Some embodiments of the method may also comprise selecting one of the subsets for further statistical analysis based on the comparison of the projection scores.

In some embodiments, comparing the projection scores may be part of a statistical hypothesis test.

According to some embodiments, the subset S may be predetermined. In other embodiments, a step of selecting the subset S may be included. The selection may e.g. be an automated selection, for instance based on the supported dimensionality of the underlying data. Alternatively or additionally, the step of selecting S may comprise prompting a user (e.g. through a man-machine interface, such as a computer monitor) for a selection of S or other input data from which S can be determined. The step of selecting S may then further comprise receiving signals representative of said user selection of S, or representative of said input data from which S can be determined (e.g. through a man-machine interface, such as a keyboard, computer mouse, or the like)

It is an advantage of embodiments of the present invention that a relatively fast computer-aided analysis of relatively large sets of technical measurement data, for selecting relevant subsets thereof, is facilitated. According to some embodiments of the present invention, the expectation values $\mathbb{E}_{\mathcal{P}_{\phi_m(X)}}\left[g(\Lambda_{\phi_m(X)}, S)\right]$ may be precomputed provided that e.g. the null distributions $P_{\phi_m(X)}$ are known or estimated on beforehand, whereby an even further improved computational speed is facilitated. The analysis may be performed with relatively modest computer hardware for relatively large sets of data. This should be compared with other known analysis methods, which may require computers with significantly higher computational capabilities to carry out the analysis. Hence, a technical effect associated with embodiments of the present invention is that it can be performed using relatively modest computer hardware. Another technical effect, due to the relatively low computational complexity, is that the analysis may be performed at a relatively low energy consumption.

Embodiments of the invention may be embedded in a computer program product, which enables implementation of the method and functions described herein. Said embodiments of the invention may be carried out when the computer program product is loaded and run in a system having computer capabilities, such as a computer 200 schematically illustrated in Fig. 11. Computer program, software program, program product, or software, in the present context mean any expression, in any programming language, code or notation, of a set of instructions intended to cause a system having a processing capability to perform a particular function directly or after conversion to another language, code or notation. The computer program product may be stored on a computer-readable medium 300, as schematically illustrated in Fig. 11. The computer 200 may be configured to perform one or more embodiments of the method. The computer 200 may e.g. be configured by loading the above-mentioned computer program product into a memory of the computer 200, e.g. from the computer-readable medium 300 or from some other source.

Although embodiments of the method described above have been limited to analyzing technical measurement data, the analysis method may, in other embodiments, also be applied to analyzing any kind of multivariate data, e.g. also to data arising from fields traditionally considered as non-technical fields, such as but not limited to financial data. Accordingly, in some embodiments, there is provided a computer-implemented method for analyzing multivariate data. Furthermore, in some embodiments, there is provided a computer-implemented method for analyzing technical measurement data. Regardless of which, the technical effects described above relating to computational speed, possibility of carrying out the analysis with relatively modest computer hardware, and/or possibility of carrying out the analysis at a relatively low energy consumption, are attainable in embodiments of the present invention.

The method of analyzing multivariate data may, in some embodiments, be used as a step in a method of determining relationships between a plurality of parameters, such as physical parameters, biological parameters, or a combination thereof. For example, multivariate data representing multiple samples of measured (or observed) values of said plurality of parameters may first be obtained (e.g. through measurement and/or retrieval from a database). Said multivariate data may then be analyzed using the above-described method of analyzing multivariate data. Advantages of utilizating of the method of analyzing multivariate data in the context of determining relationships between a plurality of physical and/or biological parameters are the same as stated above, relating to computational speed, possibility of carrying out the analysis with relatively modest computer hardware, and/or possibility of carrying out the analysis at a relatively low energy consumption. In addition thereto, utilization of the method of analyzing multivariate data in the context of determining relationships between a plurality of physical and/or biological parameters has the advantage that it facilitates discovery of relationships that might have been missed using other analysis methods (see e.g. the discussion under "Example 1" above). The relationship to be determined may e.g. be a relationship between the occurrence of a certain biological condition (e.g. a disease or the like) and certain physical and/or biological parameters (which may e.g. be represented, at least in part, by Omics data).

The present invention has been described above with reference to specific embodiments. However, other embodiments than the above described are possible within the scope of the invention. Different method steps than those described above, may be provided within the scope of the invention. The different features and steps of the embodiments may be combined in other combinations than those described. The scope of the invention is only limited by the appended patent claims.

**Claims**

1. A computer-implemented method for analyzing multivariate data comprising a plurality of samples of each of a plurality of measurement variables, comprising
   for a first subset $\phi_A(X)$ of the multivariate data $X$, determining (110) a first projection score related to the first subset;
   for a second subset $\phi_B(X)$ of the multivariate data $X$, determining (120) a second projection score related to the second subset; and
   comparing (130) the first and the second projection score for determining which one of the first and the second subset provides the most informative representation of the multivariate data, which is defined as the one of said subsets having the highest related projection score; wherein
   the projection score related to a given submatrix $\phi_m(X)$ of the multivariate data matrix $X$ is defined as

$$\sigma\big(\phi_m(X), S, \mathcal{P}_{\phi_m(X)}\big) = \frac{g(\Lambda_{\phi_m(X)}, S)}{\mathbb{E}_{\mathcal{P}_{\phi_m(X)}}[g(\Lambda_{\phi_m(X)}, S)]},$$

   wherein

- $\phi_m(X)$ is a $K_m \times N$ matrix of rank $r$ comprising measurement data of the subset, wherein $K_m$ and N are integers representing the number of variables and the number of samples, respectively;
- $g(\Lambda_{\phi_m(X)}, S)$ is selected from the set

$$G = \{h \circ \alpha_q : \mathbb{R} \to \mathbb{R} \ ; h \text{ is increasing and } q \geq 1\}$$
for

$$\alpha_q\left(\Lambda_{\phi_{m(X)}}, S\right) = \frac{\sum_{k \in S} \lambda_k^q\left(\phi_m(X)\right)}{\sum_{k=1}^{r} \lambda_k^q\left(\phi_m(X)\right)}$$

- $\lambda_1 \geq \lambda_2 \geq ... \geq \lambda_r > 0$ are the singular values of $\phi_m(X)$;
- S is a set of indices i representing principal components of $\phi_m(X)$ onto which the data in $\phi_m(X)$ is projected;and
-

$$\mathbb{E}_{\mathcal{P}_{\phi m(X)}}\left[g(\Lambda_{\phi_{m(X)}}, S)\right]$$

is the expectation value, or estimate thereof, of $g(\Lambda_{\phi_{m(X)}}, S)$ for a matrix probability distribution $P_{\phi m(X)}$.

2. The method according to claim 1, comprising:

   for each of one or more additional subsets of the multivariate data, determining a projection score related to that subset.

3. The method according to claim 2, comprising:

   comparing the projection scores related to the one or more additional subsets of the multivariate data and the first and the second projection score for determining which one of the first and the second subset provides the most informative representation of the multivariate data, which is defined as the one of said subsets having the highest related projection score.

4. The method according to any preceding claim, further comprising selecting one of the subsets for further statistical analysis based on the comparison of the first and the second projection score.

5. The method according to any of the claims 1-3, wherein the comparing the projection scores is part of a statistical hypothesis test.

6. The method according to any preceding claim, wherein the multivariate data is technical measurement data.

7. The method according to claim 6, wherein the measurement data is astronomical measurement data.

8. The method according to claim 6, wherein the measurement data is meteorological measurement data.

9. The method and according to claim 6, wherein the measurement data is biological measurement data.

10. A computer program product comprising computer program code means for executing the method according to any of the claims 1 - 9 when said computer program code means are run by an electronic device (200) having computer capabilities.

11. A computer readable medium (300) having stored thereon a computer program product comprising computer program code means for executing the method according to any of the claims 1 - 9 when said computer program code means are run by an electronic device (200) having computer capabilities.

**12.** A computer (200) configured to perform the method according to any of the claims 1-9.

**13.** A method of determining a relationship between a plurality of physical and/or biological parameters, comprising

- obtaining multivariate data representing multiple samples of observed values of said plurality of parameters; and
- analyzing the multivariate data using the method according to any of the claims 1-9.

Fig. 1

Fig. 2

Fig. 3

Fig. 4

Fig. 5

Fig. 6

Fig. 7

Fig. 8

Fig. 9

START
100

determine first projection score

110

determine second projection score

120

compare projection scores

130

END
140

Fig. 10

300

200

Fig. 11

**EP 2 434 411 A1**

<table>
<tr><td>Europäisches Patentamt<br>European Patent Office<br>Office européen des brevets</td><td style="text-align:center">**EUROPEAN SEARCH REPORT**</td><td>Application Number<br>EP 10 18 0086</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Magnus Fontes: "Statistical and knowledge supported visualization of multivariate data",<br><br>,<br>31 August 2010 (2010-08-31), XP55002519,<br>Retrieved from the Internet:<br>URL:http://arxiv.org/PS_cache/arxiv/pdf/10 08/1008.5374v1.pdf<br>[retrieved on 2011-07-11]<br>* page 11, definition 2.2 *<br>* sections 5 and 7.1 *<br>----- | 1-13 | INV.<br>G06F17/18 |

| TECHNICAL FIELDS SEARCHED (IPC) |
|---|
| G06F |

The present search report has been drawn up for all claims

| Place of search | Date of completion of the search | Examiner |
|---|---|---|
| Berlin | 15 July 2011 | Virnik, Elena |

EPO FORM 1503 03.82 (P04C01)